# EUROPEAN PATENT SPECIFICATION

(11) **EP 4 358 931 B1**
(45) Date of publication and mention of the grant of the patent: **09.04.2025**
(21) Application number: 22728254.8
(22) Date of filing: 11.05.2022
(51) Int. Cl.: A61K 8/55, A61K 8/34, A61K 8/36, A61K 8/37, A61K 8/39, A61K 8/49, A61K 8/86, A61Q 15/00

(54) **AN ANTIPERSPIRANT COMPOSITION**
SCHWEISSHEMMENDE ZUSAMMENSETZUNG
COMPOSITION ANTITRANSPIRATION

(30) Priority: 21.06.2021 WO PCT/CN2021/101152; 23.07.2021 EP 21187344
(43) Date of publication of application: 01.05.2024
(73) Proprietor: Unilever IP Holdings B.V., 3013 AL Rotterdam (NL); Unilever Global IP Limited, Wirral, Merseyside CH62 4ZD (GB)
(72) Inventor: LI, Xiaoke, 6708 WH Wageningen (NL); WANG, Weichong, 6708 WH Wageningen (NL)
(74) Representative: Whaley, Christopher
(86) International application number: PCT/EP2022/062717
(87) International publication number: WO 2022/268403

(56) References cited:
- EP-A1- 2 881 104
- CN-A- 104 887 550
- US-A1- 2012 009 136

## Description

### Field of the invention

The present invention is in the field of antiperspirant compositions.

### Background of the invention

The present invention relates to compositions that contain antiperspirant actives. These actives are added to compositions to reduce perspiration upon topical application of the compositions to the body, particularly to the underarm regions of the human body viz. the axilla, and sometimes even on the upper part of the body near the chest.

Usually, conventional antiperspirant actives are salts of certain metals having an astringent effect, such as the salts of aluminium and/or zirconium. Since antiperspirants are used regularly, and have been used for decades, there is an ever-increasing need to develop alternative antiperspirant actives which are equally efficacious.

Antiperspirant compositions characterised by contents of phospholipids are disclosed in EP 2 881 104 A1, CN 104 887 550 A, and US 2012/009136 A1.

### Summary of the invention

The present inventors have surprisingly observed that the combination of certain amount of lecithin, a specific surfactant can be stable with the presence of a specific organic compound in a composition with the low amount of water. In addition to being stable, such combination is capable of forming aggregation/agglomeration when they contact with aqueous saline media, which is equivalent to perspiration or sweat, thereby is capable of preventing sweat from rapidly being released. In this manner, such combination behaves much like conventional antiperspirants.

In accordance with a first aspect is disclosed an antiperspirant composition comprising:
(i) lecithin;
(ii) a non-ionic surfactant having a HLB value lower than 4.7 or higher than 8.6; and
(iii) at least one organic compound selected from fatty acid having 10-22 carbons, and fatty alcohol which is selected from oleyl alcohol, isostearyl alcohol, lanolin alcohol, heptanol, octanol, decanol, and nonanol; and
(iv) a cosmetically acceptable carrier;
wherein said composition comprises less than 30% of water; wherein said composition comprises at least 3% of said lecithin by weight of the composition.

In accordance with a second aspect is disclosed a non-therapeutic method of reducing perspiration comprising a step of topical application of the composition of the first aspect on to the desired skin surface.

In accordance with a third aspect is disclosed non-therapeutic use of the composition of the first aspect for reduction of bodily perspiration.

All other aspects of the present invention will more readily become apparent upon considering the detailed description and examples which follow.

Except in the examples, or where otherwise explicitly indicated, all numbers in this description indicating amounts of material or conditions of reaction, physical properties of materials and/or use may optionally be understood as modified by the word "about".

All amounts are by weight of the antiperspirant composition, unless otherwise specified. It should be noted that in specifying any ranges of values, any particular upper value can be associated with any particular lower value.

For the avoidance of doubt, the word "comprising" is intended to mean "including" but not necessarily "consisting of" or "composed of". In other words, the listed steps or options need not be exhaustive.

Where a feature is disclosed with respect to a particular aspect of the invention (for example a composition of the invention), such disclosure is also to be considered to apply to any other aspect of the invention (for example a method of the invention) *mutatis mutandis.*

### Detailed description of the invention

By "An antiperspirant Composition" as used herein, is meant to include a composition for topical application to the skin of mammals, especially humans. Such a composition is preferably of the leave-on type. By a leave-on composition is meant a composition that is applied to the desired skin surface and left on for one minute to 24 hours after which it may be wiped or rinsed off with water, usually during the regular course of personal washing. The composition may also be formulated into a product which is applied to a human body for improving the appearance, cleansing, odor control or general aesthetics. The composition of the present invention can be in the form of a liquid, lotion, cream, gel or stick form and may be delivered through a roll-on device or using an aerosol can which contains a propellant. "Skin" as used herein is meant to include skin on any part of the body (e.g., neck, chest, back, arms, underarms, hands, legs, buttocks and scalp) especially the underarms.

### HLB value

"HLB value" refers to a value defining the affinity of a surfactant for water or oil. HLB numbers can be calculated for nonionic surfactants, and these surfactants have numbers ranging from 0-20.

### Lecithin

Lecithin is a generic term to designate any group of yellow-brownish fatty substances occurring in animal and plant tissues which are amphiphilic. Lecithins are mixtures of glycerophospholipids including phosphatidylcholine, phosphatidylethanolamine, phosphatidylinositol, phosphatidylserine, and phosphatidic acid.

Commercial lecithin is a mixture of various phospholipids, and the composition depends on the origin of the lecithin. It is usually available from sources such as egg yolk, marine sources, soybeans, milk, rapeseed, cottonseed, and sunflower oil. It can be soybean-derived lecithin, sunflower-derived lecithin, or egg yolk-derived lecithin.

Antiperspirant compositions in accordance with this invention comprise at least 3% of the lecithin, preferably from 3 to 80 wt%, and more preferably from 5 to 40 wt%, and furthermore preferably from 5 to 20 wt% of the lecithin, based on total weight of the composition.

### Surfactant

Antiperspirant composition in accordance with this invention comprises a surfactant having a HLB value lower than 4.7 or higher than 8.6.

It is preferred that the surfactant is a non-ionic surfactant having a HLB value from 1 to 4.5, or from 9 to 20.

Preferably, the non-ionic surfactant can be selected from sorbitan ester, polyol ester, ethoxylated sorbitan ester, ethoxylated triglyceride, ethoxylated glyceryl ester and ester of ethoxylated sorbitol.

It is preferred that the non-ionic surfactant having a HLB value from 1 to 4.5 is selected from sorbitan ester and polyol ester. The sorbitan ester can be selected from sorbitan trioleate, sorbitan oleate and sorbitan sesquioleate. The polyol ester can be selected from glyceryl monooleate, glyceryl dioleate, triolein, polyglyceryl-2 triisostearate, polyglyceryl-2 dioleate, poyglyceryl ricinoleate, polyglyceryl-10 decaoleate, polyglyceryl-2 tetraisostearate, propylene glycol isostearate, glyceryl isostearate, caprylin and glyceryl ricinoleate.

Alternatively, it preferred that the non-ionic surfactant having a HLB value from 9 to 20 is selected from ethoxylated sorbitan ester, ethoxylated triglyceride, ethoxylated glyceryl ester, ester of ethoxylated sorbitol and polyol ester. The ethoxylated sorbitan ester can be selected from polysorbate 20, polysorbate 21, PEG-80 sorbitan laurate, polysorbate 80, polysorbate 81 and polysorbate 85. The ethoxylated triglyceride can be selected from PEG-25 hydrogenated castor oil, PEG-29 castor oil, PEG-35 castor oil and PEG-40 castor oil. The ethoxylated glyceryl ester can be selected from PEG-6 caprylic/capric glycerides, PEG-7 glyceryl cocoate and PEG-45 palm kernel glycerides. The ester of ethoxylated sorbitol can be selected from PEG-40 sorbitan peroleate, sorbeth-40 hexaoleate and sorbeth-50 hexaoleate. The polyol ester can be selected from PEG-8 dilaurate, PEG-8 laurate, PEG-20 glyceryl triisostearate, polyglyceryl-3 polyricinoleate, polyglyceryl-6 oleate, polyglyceryl-8 oleate, polyglyceryl-10 oleate, polyglyceryl-10 dioleate, polyglyceryl-4 laurate, polyglyceryl-6 laurate, polyglyceryl-10 laurate, polyglyceryl-10 dilaurate, polyglyceryl-3 octoate, polyglyceryl-6 octoate, polyglyceryl-10 octoate, polyglyceryl-6 ricinoleate, polyglyceryl-10 ricinoleate, polyglyceryl-10 isostearate, polyglyceryl-10 diisostearate, polyglyceryl-10 decanoate, polyglyceryl-6 decanoate, polyglyceryl-3 decanoate.

More preferably, the non-ionic surfactant is selected from sorbitan trioleate, triolein, polyglyceryl-2 triisostearate, poyglyceryl ricinoleate, polyglyceryl-2 tetraisostearate, propylene glycol isostearate, glyceryl isostearate, polysorbate 20, polysorbate 80, PEG-45 palm kernel glycerides, polyglyceryl-10 laurate, polyglyceryl-6 octoate, polyglyceryl-10 octoate, polyglyceryl-10 decanoate.

Antiperspirant composition in accordance with this invention preferably comprises from 3 to 80 wt%, and more preferably from 5 to 40 wt%, and furthermore preferably from 5 to 20 wt% of the surfactant, based on total weight of the composition.

It is preferred that the composition in accordance with this invention comprises the lecithin and the surfactant in a weight ratio of from 1:10 to 10:1, preferably from 1:5 to 5:1, and most preferably from 1:3 to 3:1.

### Organic compound

Antiperspirant composition in accordance with this invention additionally comprises at least one organic compound selected from fatty acid having from 10 to 22 carbons and fatty alcohol which is selected from oleyl alcohol, isostearyl alcohol, lanolin alcohol, heptanol, octanol, decanol, and nonanol..

When the organic compound is a fatty acid having from 10 to 22 carbons, it is preferred to be unsaturated fatty acid. The fatty acid can be selected from oleic acid, linoleic acid, ricinoleic acid, isostearic acid, palmitoleic acid, arachidonic acid, timnodonic acid, docosahexaenoic acid, octanoic acid, vaccenic acid, erucic acid.

When the organic compound is a fatty alcohol, it is selected from oleyl alcohol, isostearyl alcohol and lanolin alcohol, heptanol, octanol, decanol, nonanol.

It is preferred that the antiperspirant compositions in accordance with this invention comprises from 3 to 80 wt%, more preferably from 20 to 70 wt%, furthermore preferably from 30 to 60 wt% of the organic compound, based on total weight of the composition.

The weight ratio of organic compound to the combination of the lecithin and the surfactant is preferred to be from 1:10 to 10:1, more preferred to be from 1:5 to 5:1, and most preferred to be from 1:3 to 3:1.

Without wishing to be bound by theory it is believed that the combination of certain amount of lecithin and a specific surfactant with the presence of a specific organic compound of the present invention can be stable in the composition with low amount of water. In addition to being stable, such combination is capable of forming aggregation/agglomeration when they contact with aqueous saline media, which is equivalent to perspiration or sweat, thereby is capable of preventing sweat from rapidly being released.

Antiperspirant compositions in accordance with this invention may advantageously comprise an additional antiperspirant active. However, the composition of the present invention is preferably free from antiperspirant active which comprises aluminium or zirconium. And the composition of the present invention is preferably free from antiperspirant active which is zinc based.

Antiperspirant active which comprises aluminium or zirconium is preferably selected from aluminium/zirconium halides and halohydrate salts, aluminum-zirconium tetrachlorohydrex glycine complex, aluminum-zirconium octachlorohydrex glycine complex, aluminum-zirconium pentachlorohydrate, aluminum sesquichlorohydrate or mixtures thereof, more preferably aluminum sesquichlorohydrate or aluminium chlorohydrates. Antiperspirant active which comprises aluminium or zirconium can also be complexes based on the above-mentioned astringent aluminium and/or zirconium salts and the complex often employs a compound with a carboxylate group, and advantageously this is an amino acid.

Zinc based antiperspirant active is preferably selected from zinc oxide, zinc hydroxide, zinc hydroxide ions with counter ions, and zinc ions with counter ions. Counter ions may include halides and amino acid salt.

Non- aluminum or zirconium, or non-zinc antiperspirant active is preferably selected from glycerol monolaurate plus isostearyl alcohol, chitosan or a salt thereof with a weight average molecular weight of from 250 to 650 kDa, titanium compound chelated by alkanolamine with an acid and a polyhydric alcohol, or cholic acid derivative selected from a hydroxycholic acid or a salt thereof with a multivalent metal salt.

More preferably, the composition of the present invention is free from metal based antiperspirant active. The metal includes aluminum, zirconium, zinc, titanium, copper, gallium, stannum, Indium, hafnium, vanadium, cobalt.

### pH of compositions

It is preferred that pH of the antiperspirant composition of the present invention is preferably from 2 to 9, more preferably 3 to 7.

### Other ingredients

Other components commonly included in conventional antiperspirant compositions may also be incorporated in the compositions of the present invention. Such components include skin care agents such as emollients, humectants and skin barrier promoters; skin appearance modifiers such as skin lightening agents and skin smoothing agents; anti-microbial agents, in particular organic anti-microbial agents, and preservatives.

The antiperspirant compositions of the invention are applied cosmetically and topically to the skin, broadly speaking, by one of two methods. Different consumers prefer one method or the other. In one method, sometimes called a contact method, the composition is wiped across the surface of the skin, depositing a fraction of the composition as it passes. In the second method, sometimes called the non-contact method, the composition is sprayed from a dispenser held proximate to the skin, often in an area of about 10 to 20 cm². The spray can be developed by mechanical means of generating pressure on the contents of a dispenser, such as a pump or a squeezable sidewall or by internally generated pressure arising from a fraction of a liquefied propellant volatilizing, the dispenser commonly being called an aerosol.

There are broadly speaking two classes of contact compositions, one of which is liquid and usually applied using a roll-on dispenser or possibly absorbed into or onto a wipe, and in the second of which the antiperspirant active is distributed within a carrier liquid that forms a continuous phase that has been gelled. In one variation, the carrier fluid comprises a solvent for the antiperspirant and in a second variation, the antiperspirant remains a particulate solid that is suspended in an oil, usually a blend of oils.

The composition of the invention comprises cosmetically acceptable carrier. The composition in accordance with this invention comprises less than 30% of water, preferably less than 15%, furthermore preferably less than 10% by weight of the composition.

It is preferred that the weight ratio of the amount of the combination of the lecithin and the surfactant, and the amount of water is higher than 1, preferably higher than 3, more preferably higher than 4.

It is preferred that the composition of the invention is in the form of lotion, spray, firm solid, soft solid, cream, or is an emulsion packaged in a roll-on applicator.

It is preferred that, when said composition is a spray it comprises a propellant and the composition is in the form of an aerosol.

### Stick compositions

In one aspect, the antiperspirant composition of the invention is a stick composition.

Antiperspirant sticks can be formulated as clear (i.e., translucent or transparent) or opaque compositions. Translucent or transparent sticks go on clear and, depending upon their formulation, may remain clear for extended periods of time, reducing the consumer perceived negative of "white marks" associated with deposition of antiperspirant active. Many different materials have been proposed as gellants for a continuous oil phase, including waxes, small molecule gelling agents and polymers. They each have their advantages and of them, one of the most popular class of gellants is waxes, partly at least due to their ready availability and ease of processing, including in particular linear fatty alcohol wax gellants. A gelled antiperspirant composition is applied topically to skin by wiping it across and in contact with the skin, thereby depositing on the skin a thin film.

The nature of the film depends to a significant extent on the gellant that is employed. Although fatty alcohols have been employed as gellants for many years, and are effective for the purpose of gelling, the resultant product is rather ineffective at improving the visual appearance of skin, and in particular underarm skin, to which the composition has been applied. This problem has been solved by including ameliorating materials for example, di or polyhydric humectants and/or a triglyceride oil.

Stick compositions are usually available in the form of a firm solid or a soft solid. Firm solids, as the name indicates, are harder and can be directly applied by way of an applicator, for example, to the underarms. Soft solids also need an applicator which is similar to the firm solids, the difference being that the soft solids are softer and the applicator needs to be designed in order to permit extrusion of the solids through a cap member comprising plurality of orifices and the extruded composition can then be applied to the underarms.

### Roll-on

Liquid compositions that are applicable from a roll-on broadly speaking can be divided into two classes, namely those in which an antiperspirant active is suspended in a hydrophobic carrier, such as a volatile silicone and those in which the antiperspirant active is dissolved in a carrier liquid. The latter has proven to be more popular. There are mainly two sorts of dissolving carrier liquid, namely carriers that are predominantly alcoholic, which is to say the greater part of the dissolving carrier fluid comprises ethanol and the second class in which the carrier liquid is mainly water. The former was very popular because ethanol is a mild bactericide in its own right, but its popularity waned because it stings, especially if the surface onto which the composition has been applied has been damaged or cut, such as can easily arise during shaving or other depilatory actions.

Alternatively, the composition of the invention is a liquid composition, that can be dispensed from a roll-on package. Broadly speaking such compositions could be divided into two classes, namely those in which an antiperspirant active is suspended in a hydrophobic carrier, such as a volatile silicone and those in which the antiperspirant active is dissolved in a carrier liquid, such as glycerin and propylene glycol.

The second class of formulations that is an alternative to alcoholic formulations comprise a dispersion of water-insoluble or very poorly water-soluble ingredients in an aqueous solution of the antiperspirant. Herein, such compositions will be called emulsions. Antiperspirant roll-on emulsions commonly comprise one or more emulsifiers to maintain a distribution of the water-soluble ingredients.

### Aerosol compositions

Further alternatively, the antiperspirant composition of the invention is delivered through an aerosol composition which comprises a propellant in addition to the applicable other ingredients described hereinabove. Commonly, the propellant is employed in a weight ratio to the base formulation of from 99:1 to 1:99. Depending on the propellant, in such aerosol compositions the ratio of propellant to base formulation is normally at least 20:80, generally at least 30:70, particularly at least 40:60, and in many formulations, the weight ratio is from 90:10 to 50:50. A ratio range of from 70:30 to 90:10 is sometimes preferred.

Propellants herein generally are one of three classes; (i) low boiling-point gasses liquified by compression, (ii) volatile ethers and (iii) compressed non-oxidising gases.

Class (i) is conveniently a low boiling-point material, typically boiling below -5°C, and often below -15°C, and in particular, alkanes and/or halogenated hydrocarbons. This class of propellant is usually liquefied at the pressure in the aerosol canister and evaporates to generate the pressure to expel the composition out of the canister. Examples of suitable alkanes include particularly propane, butane or isobutane. The class (ii) of propellant comprises a very volatile ether of which the most widely employed ether hitherto is dimethyl ether. This propellant can advantageously be employed at relatively low weight ratio of propellant to base formulation, for example to as low as 1:99. It can also be employed in admixture with, for example, compressible/liquefiable alkane gasses. The class (iii) of propellant comprises compressed non-oxidising gasses, and in particular carbon dioxide or nitrogen. Inert gases like neon are a theoretical alternative.

The composition of the invention comprises a topically acceptable carrier. When the composition of the invention is in the form of a roll-on, a cream, a spray, or a firm solid or a soft solid, the topically acceptable carrier comprises an anhydrous carrier or an aqueous carrier. The anhydrous carrier in such cases may comprise a silicone compound, low boiling alcohol or a wax. When the composition comprises a propellant, it is delivered as an aerosol.

The composition of the present invention can comprise a wide range of other optional components. The CTFA Personal Care Ingredient Handbook, Second Edition, 1992, describes a wide variety of non-limiting personal care and pharmaceutical ingredients commonly used in the skin care industry, which are suitable for use in the compositions of the present invention. Examples include: antioxidants, binders, biological additives, buffering agents, colorants, thickeners, polymers, astringents, fragrance, conditioners, exfoliating agents, pH adjusters, other than the ones already discussed earlier, preservatives, natural extracts, essential oils, skin sensates, skin soothing agents, and skin healing agents.

A preservative is a preferred additional component in compositions of the invention. A preservative serves to reduce or eliminate microbial contamination of compositions of the invention. Preservatives are typically employed at a total level of from 0.05 to 3%, preferably at from 0.1 to 2% and most preferably at from 0.4 to 1%.

Suitable preservatives for use with the present invention include 2-phenoxyethanol, polylysine, iodopropynyl butylcarbamate, C₁-C₃ alkyl parabens, sodium benzoate, caprylyl glycol and EDTA. Particularly preferred preservatives are 2-phenoxyethanol, iodopropynyl butylcarbamate, sodium benzoate, caprylyl glycol and EDTA and especially preferred are 2-phenoxyethanol and iodopropynyl butylcarbamate.

A preferred additional component of compositions of the invention is a fragrance. Suitable materials include conventional perfumes, such as perfume oils and also include so-called deo-perfumes, as described in EP 545,556 and other publications. Levels of incorporation are preferably up to 4% by weight, particularly from 0.1% to 2% by weight, and especially from 0.7% to 1.7% by weight.

An antimicrobial deodorant active is a preferred an additional component in compositions of the invention. Such components serve to reduce or eliminate body odour by reducing or otherwise impeding the function of microbes on the skin of the body responsible for malodour generation.

The antimicrobial deodorant active may also be a preservative for the composition.

When employed, the anti-microbial deodorant agent is typically incorporated into the composition at from 0.01% to 3% and particularly at from 0.03% to 0.5%.

Preferred anti-microbial deodorant agents have a minimum inhibitory concentration (MIC) of 1 mg.ml⁻¹ or less, particularly 200 µg.ml⁻¹ or less, and especially 100 µg.ml⁻¹ or less. The MIC of an anti-microbial agent is the minimum concentration of the agent required to significantly inhibit microbial growth. Inhibition is considered "significant" if an 80% or greater reduction in the growth of an inoculum of *Staphylococcus epidermidis* is observed, relative to a control medium without an anti-microbial agent, over a period of 16 to 24 hours at 37°C. Details of suitable methods for determining MICs can be found in "Antimicrobial Agents and Susceptibility Testing", C.Thornsberry, (in "Manual of Clinical Microbiology", 5th Edition, Ed. A. Balows et al, American Society for Microbiology, Washington D.C., 1991). A particularly suitable method is the Macrobroth Dilution Method as described in Chapter 110 of above publication (pp. 1101-1111) by D. F. Sahm and J. A. Washington II. MICs of anti-microbials suitable for inclusion in the compositions of the invention are triclosan: 0.01-10 µg.ml⁻¹ (J.Regos et al., Dermatologica (1979), 158: 72-79) and farnesol: ca. 25 µg.ml⁻¹ (K. Sawano, T. Sato, and R. Hattori, Proceedings of the 17th IFSCC International Conference, Yokahama (1992) p.210-232). By contrast ethanol and similar alkanols have MICs of greater than 1 mg.ml⁻¹.

Suitable organic anti-microbials are bactericides, for example quaternary ammonium compounds, like cetyltrimethylammonium salts; chlorhexidine and salts thereof; and diglycerol monocaprate, diglycerol monolaurate, glycerol monolaurate, and similar materials, as described in "Deodorant Ingredients", S.A.Makin and M.R.Lowry, in "Antiperspirants and Deodorants", Ed. K. Laden (1999, Marcel Dekker, New York). More preferred anti-microbials for use in the compositions of the invention are polyhexamethylene biguanide salts (also known as polyaminopropyl biguanide salts), an example being Cosmocil CQ^{™} available from Zeneca PLC, preferably used at up to 1% and more preferably at 0.03% to 0.3% by weight; 2',4,4'-trichloro,2-hydroxy-diphenyl ether (triclosan), preferably used at up to 1% by weight of the composition and more preferably at 0.05-0.3%; and 3,7,11-trimethyldodeca-2,6,10-trienol (farnesol), preferably used at up to 1% by weight of the composition and more preferably at up to 0.5%.

Other suitable organic antimicrobial agents are transition metal chelators, as described in WO01/52805, for example. Transitional metal chelators having a binding coefficient for iron(III) of greater than 10²⁶, for example diethylenetriaminepentaacetic acid and salts thereof are preferred.

### Method and Use

The present invention also provides for a method of reducing perspiration comprising a step of topical application of the composition of the first aspect. Preferably, the present invention provides for a method wherein the composition of the first aspect is applied on the underarms. The present invention also provides for a method wherein topical application of the composition of the first aspect reduces perspiration from the surface of the human body. The method in accordance with the invention is non-therapeutic. By non-therapeutic is meant that the method is cosmetic in nature.

The invention also provides for use of the composition of the first aspect for reduction of bodily perspiration. The use in accordance with the invention is non-therapeutic in nature, more preferably cosmetic in nature.

The invention will now be demonstrated with the help of the following non-limiting examples.

### Examples

### Materials listed in Table -1 were put through some tests as detailed hereinafter.

**Table -1**

| **Trade name** | **Chemical ingredient** | **Supplier** | **HLB value** |
|---|---|---|---|
| Lecithin | Phosphatidylcholine | Lipoid Kosmetik | - |
| Span85 | Sorbitan trioleate | Sinopharm Chemical Reagent Co. Ltd. | 1.8 |
| Triolein | Glyceryl trioleate | Shandong Binzhou GIN&ING New Material Technology Co.Ltd. | 2.5 |
| Tween80 | Polyoxyethylene sorbitan monooleate | Sinopharm Chemical Reagent Co.Ltd. | 15 |
| Span120 | Sorbitan Isostearate | Croda | 4.7 |
| Span20 | Sorbitan Laurate | Croda | 8.6 |
| Oleic acid | Oleic acid | Sinopharm Chemical Reagent Co.Ltd. | - |
| Oleyl alcohol | Oleyl alcohol | TCI (Shanghai) Development Co., Ltd. | |
| Ethanol | Ethanol | Sinopharm Chemical Reagent Co.Ltd. | - |

### Examples

This example demonstrated the composition comprises the combination of certain amount of lecithin and specific surfactant with the presence of the specific organic compound of the present invention is capable of forming aggregation/agglomeration when they contact with aqueous saline media. All ingredients are expressed by weight percent of the total formulation, and as level of active ingredient.

The compositions indicated in Table -2 were prepared.

**Table -2**

| Ingredien t % | | | **Samples** | | | | | | | | |
|---|---|---|---|---|---|---|---|---|---|---|---|
| | **A** | **B** | **C** | **D** | **E** | **1** | **2** | **3** | **4** | **5** | **6** |
| Lecithin | 2.7 | 18.8 | 17.4 | 18.8 | 18.8 | 7.3 | 18.8 | 21.2 | 18.8 | 18.8 | 18.8 |
| Oleic acid | 4.5 | - | 34.8 | 31.3 | 31.3 | 12.2 | 31.3 | 42.4 | 31.3 | 31.3 | - |
| Oleyl alcohol | - | - | - | - | - | - | - | - | - | - | 31.3 |
| Span85 | 2.7 | 18.8 | 17.4 | - | - | 7.3 | 18.8 | 21.2 | - | - | 18.8 |
| Span120 | - | - | - | 18.8 | - | - | - | - | - | - | - |
| Span20 | - | - | - | - | 18.8 | - | - | - | - | - | - |
| Triolein | - | - | - | - | - | - | - | - | 18.8 | - | - |
| Tween80 | - | - | - | - | - | - | - | - | - | 18.8 | - |
| Water | - | - | 30.4 | - | - | - | - | 15.2 | - | - | - |
| Ethanol | To 100 | To 100 | To 100 | To 100 | To 100 | To 100 | To 100 | To 100 | To 100 | To 100 | To 100 |

The composition of model ionic sweat (pH 6.9) is as given below in Table -3:

**Table -3**

| **Ingredient** | **wt% of total** |
|---|---|
| Potassium Chloride | 0.0373 |
| Sodium Bicarbonate | 0.2025 |
| Sodium Chloride | 0.2098 |
| Ammonium Chloride | 0.0107 |
| Calcium Chloride | 0.0222 |
| Lactic Acid | 0.0901 |
| Urea | 0.0018 |
| Water | 99.4256 |

### Testing On A Narrow Capillary Apparatus

An experiment was arranged using the device as disclosed in the International Application WO2018099931 A1 (Unilever).

The device has a 20X20 µm channel. Before use, the channel was filled with model ionic sweat. The surface tension of the model ionic sweat is strong and prevents its flowing in the capillary. 20 µl of concerned composition in Table-2, was placed at one end of the channel. When the composition contacts with the model ionic sweat, the composition of the present invention is believed to turn into something like an aggregation/agglomeration. This phenomenon simulates the manner in which an antiperspirant active agent forms aggregation/agglomeration thereby provides antiperspirant benefit in the underarm region.

The whole process was monitored under optical microscope (Leica^{™} DM 2500P).

The data on the aggregation/agglomeration formation of the compositions at the end of the channel after 60 minutes is summarized in Table -4 below:

**Table-4**

| **Samples** | **A** | **B** | **C** | **D** | **E** | **1** | **2** | **3** | **4** | **5** | **6** |
|---|---|---|---|---|---|---|---|---|---|---|---|
| Aggregation /agglomeration formed | N | N | N | N | N | Y | Y | Y | Y | Y | Y |

The data in Table -4 above indicates that compositions as per the invention (Samples 1 to 6) can form an aggregation/agglomeration at the end of the 20X20 µm channel when it contacts with the model ionic sweat, which further indicates its ability to form aggregation/agglomeration when used in an antiperspirant composition, while the composition outside of the scope of the present invention (Samples A to E) does not exhibit such efficacy.

## Claims

1. An antiperspirant composition comprising:
(i) lecithin;
(ii) a non-ionic surfactant having a HLB value lower than 4.7 or higher than 8.6;
(iii) at least one organic compound selected from fatty acid having 10-22 carbons, and fatty alcohol which is selected from oleyl alcohol, isostearyl alcohol, lanolin alcohol, heptanol, octanol, decanol, and nonanol; and
(iv) a cosmetically acceptable carrier;
wherein said composition comprises less than 30 wt% of water; wherein said composition comprises at least 3 wt% of said lecithin by weight of the composition.

2. An antiperspirant composition as claimed in claim 1 wherein said surfactant has a HLB value from 1 to 4.5, or from 9 to 20.

3. An antiperspirant composition as claimed in claim 2 wherein said non-ionic surfactant is selected from sorbitan ester, polyol ester, ethoxylated sorbitan ester, ethoxylated triglyceride, ethoxylated glyceryl ester and ester of ethoxylated sorbitol.

4. An antiperspirant composition as claimed in any of claims 1 to 3 wherein said organic compound is a fatty acid selected from oleic acid, linoleic acid, ricinoleic acid, isostearic acid, palmitoleic acid, arachidonic acid, timnodonic acid, docosahexaenoic acid, octanoic acid, vaccenic acid, erucic acid.

5. An antiperspirant composition as claimed in any of claims 1 to 4 wherein the weight ratio of the amount of the lecithin to the amount of surfactant is in the range of from 1:10 to 10:1, preferably from 1:5 to 5:1, and more preferably from 1:3 to 3:1.

6. An antiperspirant composition as claimed as any of claims 1 to 5 wherein said composition comprises 3 to 80 wt% of said lecithin by weight of the composition.

7. An antiperspirant composition as claimed in any of claims 1 to 6 wherein said composition comprises 3 to 80 wt% of said surfactant by weight of the composition.

8. An antiperspirant composition as claimed in any of claims 1 to 7 wherein said composition comprises 3 to 80 wt% of said organic compound by weight of the composition.

9. An antiperspirant composition as claimed in any of claims 1 to 8 wherein said composition is in the form of lotion, spray, firm solid, soft solid, cream, or is an emulsion packaged in a roll-on applicator.

10. An antiperspirant composition as claimed in any of claims 1 to 9 wherein said composition comprises a propellant and is in the form of an aerosol.

11. A non-therapeutic method of reducing perspiration comprising a step of topical application of the composition as claimed in any of claims 1 to 10 on to the desired skin surface.

12. A method as claimed in claim 11 wherein said composition is applied on the underarms.

13. Non-therapeutic use of a composition as claimed in any of claims 1 to 10 for reduction of bodily perspiration.

## Patentansprüche

1. Schweißhemmende Zusammensetzung, umfassend:
(i) Lecithin;
(ii) ein nicht-ionisches Tensid mit einem HLB-Wert von weniger als 4,7 oder mehr als 8,6;
(iii) mindestens eine organische Verbindung, ausgewählt unter einer Fettsäure mit 10 - 22 Kohlenstoffatomen und einem Fettalkohol, der aus Oleylalkohol, Isostearylalkohol, Lanolinalkohol, Heptanol, Octanol, Decanol und Nonanol ausgewählt ist; und
(iv) einen kosmetisch akzeptablen Träger;
wobei die Zusammensetzung weniger als 30 Gew.-% Wasser umfasst; wobei die Zusammensetzung mindestens 3 Gew.-% Lecithin, bezogen auf das Gewicht der Zusammensetzung, umfasst.

2. Schweißhemmende Zusammensetzung, wie im Anspruch 1 beansprucht, wobei das Tensid einen HLB-Wert von 1 bis 4,5 oder von 9 bis 20 aufweist.

3. Schweißhemmende Zusammensetzung, wie im Anspruch 2 beansprucht, wobei das nicht-ionische Tensid unter Sorbitanestern, Polyolestern, ethoxylierten Sorbitanestern, ethoxylierten Triglyceriden, ethoxylierten Glycerylestern und Estern von ethoxyliertem Sorbitol ausgewählt ist.

4. Schweißhemmende Zusammensetzung, wie in einem der Ansprüche 1 bis 3 beansprucht, wobei die organische Verbindung eine Fettsäure ist, die unter Ölsäure, Linolsäure, Ricinolsäure, Isostearinsäure, Palmitoleinsäure, Arachidonsäure, Timnodonsäure, Docosahexaensäure, Octansäure, Vaccensäure und Erucasäure ausgewählt ist.

5. Schweißhemmende Zusammensetzung, wie in einem der Ansprüche 1 bis 4 beansprucht, wobei das Gewichtsverhältnis der Menge des Lecithins zu der Menge des Tensids in dem Bereich von 1:10 bis 10:1, bevorzugt von 1:5 bis 5:1 und bevorzugter von 1:3 bis 3:1 liegt.

6. Schweißhemmende Zusammensetzung, wie in einem der Ansprüche 1 bis 5 beansprucht, wobei die Zusammensetzung 3 bis 80 Gew.-% des Lecithins, bezogen auf das Gewicht der Zusammensetzung, umfasst.

7. Schweißhemmende Zusammensetzung, wie in einem der Ansprüche 1 bis 6 beansprucht, wobei die Zusammensetzung 3 bis 80 Gew.-% des Tensids, bezogen auf das Gewicht der Zusammensetzung, umfasst.

8. Schweißhemmende Zusammensetzung, wie in einem der Ansprüche 1 bis 7 beansprucht, wobei die Zusammensetzung 3 bis 80 Gew.-% der organischen Verbindung, bezogen auf das Gewicht der Zusammensetzung, umfasst.

9. Schweißhemmende Zusammensetzung, wie in einem der Ansprüche 1 bis 8 beansprucht, wobei die Zusammensetzung in Form einer Lotion, eines Sprays, eines harten Feststoffs, eines weichen Feststoffs oder einer Creme vorliegt oder eine Emulsion ist, die in einem Roll-on-Applikator verpackt ist.

10. Schweißhemmende Zusammensetzung, wie in einem der Ansprüche 1 bis 9 beansprucht, wobei die Zusammensetzung ein Treibmittel umfasst und in Form eines Aerosols vorliegt.

11. Nicht-therapeutisches Verfahren zur Verringerung der Schweißbildung, das einen Schritt des topischen Auftragens der Zusammensetzung, wie in einem der Ansprüche 1 bis 10 beansprucht, auf die gewünschte Hautoberfläche umfasst.

12. Verfahren, wie im Anspruch 11 beansprucht, wobei die Zusammensetzung auf die Achselhöhle aufgetragen wird.

13. Nicht-therapeutische Verwendung einer Zusammensetzung, wie in einem der Ansprüche 1 bis 10 beansprucht, zur Verringerung der Körperschweißbildung.

## Revendications

1. Composition antitranspirante comprenant :
(i) de la lécithine ;
(ii) un tensioactif non-ionique ayant un indice HLB inférieur à 4,7 ou supérieur à 8,6 ;
(iii) au moins un composé organique choisi parmi un acide gras ayant 10 à 22 atomes de carbone et un alcool gras qui est choisi parmi l'alcool oléylique, l'alcool isostéarylique, l'alcool de lanoline, l'heptanol, l'octanol, le décanol et le nonanol ; et
(iv) un véhicule acceptable en cosmétique ;
laquelle composition comprend moins de 30 % en poids d'eau ; laquelle composition comprend au moins 3 % en poids de ladite lécithine, en poids de la composition.

2. Composition antitranspirante selon la revendication 1, dans laquelle ledit tensioactif a un indice HLB de 1 à 4,5, ou de 9 à 20.

3. Composition antitranspirante selon la revendication 2, dans laquelle ledit tensioactif non-ionique est choisi parmi un ester de sorbitan, un ester de polyol, un ester de sorbitan éthoxylé, un triglycéride éthoxylé, un ester de glycéryle éthoxylé et un ester de sorbitol éthoxylé.

4. Composition antitranspirante selon l'une quelconque des revendications 1 à 3, dans laquelle ledit composé organique est un acide gras choisi parmi l'acide oléique, l'acide linoléique, l'acide ricinoléique, l'acide isostéarique, l'acide palmitoléique, l'acide arachidonique, l'acide timnodonique, l'acide docosahexénoïque, l'acide octanoïque, l'acide vaccénique, l'acide érucique.

5. Composition antitranspirante selon l'une quelconque des revendications 1 à 4, dans laquelle le rapport en poids de la quantité de lécithine à la quantité de tensioactif est situé dans la plage allant de 1/10 à 10/1, de préférence de 1/5 à 5/1, et mieux encore de 1/3 à 3/1.

6. Composition antitranspirante selon l'une quelconque des revendications 1 à 5, laquelle composition comprend 3 à 80 % en poids de ladite lécithine, en poids de la composition.

7. Composition antitranspirante selon l'une quelconque des revendications 1 à 6, laquelle composition comprend 3 à 80 % en poids dudit tensioactif, en poids de la composition.

8. Composition antitranspirante selon l'une quelconque des revendications 1 à 7, laquelle composition comprend 3 à 80 % en poids dudit composé organique, en poids de la composition.

9. Composition antitranspirante selon l'une quelconque des revendications 1 à 8, laquelle composition est sous la forme d'une lotion, d'un spray, d'un solide ferme, d'un solide mou, d'une crème, ou est une émulsion conditionnée dans un applicateur à bille.

10. Composition antitranspirante selon l'une quelconque des revendications 1 à 9, laquelle composition comprend un propulseur et est sous la forme d'un aérosol.

11. Méthode non thérapeutique pour réduire la transpiration, comprenant une étape d'application topique de la composition de l'une quelconque des revendications 1 à 10 sur la surface de peau souhaitée.

12. Méthode selon la revendication 11, dans laquelle ladite composition est appliquée sur les aisselles.

13. Utilisation non thérapeutique d'une composition de l'une quelconque des revendications 1 à 10, pour réduire la transpiration corporelle.
